**Europäisches Patentamt**

**European Patent Office**

**Office européen des brevets**

(19)

(11) Veröffentlichungsnummer : **0 094 499 B2**

(12)
# NEUE EUROPÄISCHE PATENTSCHRIFT

(45) Veröffentlichungstag der neuen Patentschrift :
**04.03.92 Patentblatt 92/10**

(51) Int. Cl.⁵ : **A01M 1/20, A61L 9/12**

(21) Anmeldenummer : **83103061.4**

(22) Anmeldetag : **28.03.83**

(54) **Verdunstungsvorrichtung für Insektizide, Duftstoffe und/oder andere flüchtige Wirkstoffe.**

(30) Priorität : **15.05.82 DE 3218480**
**15.05.82 DE 3218481**
**15.05.82 DE 8214314 U**

(43) Veröffentlichungstag der Anmeldung :
**23.11.83 Patentblatt 83/47**

(45) Bekanntmachung des Hinweises auf die
Patenterteilung :
**09.07.86 Patentblatt 86/28**

(45) Bekanntmachung des Hinweises auf die
Entscheidung über den Einspruch :
**04.03.92 Patentblatt 92/10**

(84) Benannte Vertragsstaaten :
**AT BE CH DE FR GB LI**

(56) Entgegenhaltungen :
**BE-A- 779 684**
**DE-A- 2 836 008**

(56) Entgegenhaltungen :
**DE-A- 3 106 550**
**DE-B- 2 807 424**
**FR-A- 1 231 135**
**GB-A- 432 312**
**GB-A- 2 078 112**
**US-A- 3 587 968**
**US-A- 4 161 284**

(73) Patentinhaber : **Globol-Werk GmbH**
**Postfach 1360**
**W-8858 Neuburg 1 (DE)**

(72) Erfinder : **v. Philipp, Fritz**
**Auf der Klause**
**W-8858 Neuburg/Donau (DE)**
Erfinder : **Schimanski, Georg**
**Bührener Weg 41**
**W-5800 Hagen 8 (DE)**

(74) Vertreter : **Reinhard, Skuhra, Weise**
**Postfach 44 01 51 Friedrichstrasse 31**
**W-8000 München 40 (DE)**

EP 0 094 499 B2

## Beschreibung

Die Erfindung betrifft eine Verdunstungsvorrichtung für Insektizide, Duftstoffe und/oder andere flüchtige Wirkstoffe gemäß dem Oberbegriff des Patentanspruchs 1.

Verdunstungsvorrichtungen der eingangs genannten Art sind bereits aus der DE-C-28 07 424 bekannt. Diese Verdunstungsvorrichtungen ermöglichen den Einsatz nur eines einzigen Behälters mit Wirkstoffen und erfordern zudem, daß zum Zwecke des Öffnens des Wirkstoffbehälters ein Druck auf den Behälter ausgeübt werden muß, um eine Öffnung in den hüllenförmigen Behälter einzudrücken. Bei einem derartigen hüllenförmigen bzw. kugelförmigen Behälter aus elastischem Material besteht die Möglichkeit, daß die hergestellte Öffnung durch die Rückverformung des Materials wieder verschlossen wird und damit nicht der gesamte Wirkstoff kontinuierlich aus dem Behälter austritt.

Aus der GB-A-20 78 112 ist eine Verdunstungsvorrichtung bekannt, bei der das untere Gehäuseteil eine Öffnung zum Einsetzen einer Kapsel aufweist, die ein Material zum Absorbieren des zu verdunstenden Stoffes enthält. Diese Kapsel wird in die von außen zugängliche Öffnung oder Kammer eingesetzt und steht in Anlage zu einem Trägermaterial. Die Öffnungen zur Freigabe des zu verdunstenden Stoffes liegen auf der gegenüber dem Trägermaterial abgewandten Seite zur Kapsel. Bei dieser Verdunstungsvorrichtung wird die Freigabe der zu verdunstenden Stoffe dadurch erreicht, daß ein Deckel von der Kapsel vor ihrem Einsetzen in die Verdunstungsvorrichtung entfernt wird.

Der Erfindung liegt demgegenüber die Aufgabe zugrunde, eine Verdunstungsvorrichtung gemäß dem Oberbegriff des Patentanspruchs 1 derart zu verbessern, daß das Auswechseln des Behälters vereinfacht wird und gleichzeitig das Öffnen der Behälter auf sichere oder einfache Weise gewährleistet ist.

Diese Aufgabe wird erdindungsgemäß durch den kennzeichnenden Teil des Patentanspruchs 1 gelöst. Weitere Ausgestaltungen der Verdunstungsvorrichtung ergeben sich aus den Unteransprüchen.

Die Erfindung schafft eine Verdunstungsvorrichtung, in welche mehr als ein Behälter für Wirkstoffe eingesetzt werden kann. Dadurch ist es möglich, nach dem Verbrauch der Wirkstoffe eines Behälters den nächsten Behälter zur Freigabe seiner Wirkstoffe zu öffnen. Die Öffnung der Behälter selbst erfolgt auf einfache Weise dadurch, daß der betreffende Behälter in Richtung auf seine zugeordnete Öffnereinheit verschoben wird.

Zum Auswechseln leerer Wirkstoff-Behälter ist das Gehäuse auf einfache Weise zu öffnen ; durch das Austauschen leerer Behälter durch volle Wirkstoff-Behälter ist die Vorrichtung wieder über einen langen Zeitraum einsatzfähig.

Während bei einer Ausführungsform der Verdunstungsvorrichtung ein Wirkstoffe aufnehmendes bzw. aufsaugendes Element innerhalb der zu den Behältern und Öffnereinheiten abgewandten Seite des Gehäuses angeordnet ist, wird bei einer anderen Ausführungsform dieses Element, beispielsweise in Form mehrerer einzelner Schichten, zwischen den Behältern einerseits und den Öffnereinheiten andererseits vorgesehen, wobei diese Schichten vorzugsweise an der den Öffnereinheiten zugewandten Fläche der Behälter angeordnet ist.

Bei einer weiteren Ausführungsform sind die die Wirkstoffe aufnehmenden Behälter in Bezug auf die Wandfläche durchsichtig ausgebildet, welche innerhalb von Öffnungen im Gehäuse angeordnet ist ; dadurch läßt sich leicht feststellen, ob der betreffende Behälter mit Wirkstoff aufgefüllt, teilweise leer oder vollständig leer ist.

Nach einer weiteren Ausführungsform ist die den Öffnereinheiten zugewandte Fläche bzw. Wandung des Behälters flach bzw. plan ausgebildet und besteht aus einer perforierbaren Folie, während die restliche Behälterwandung aus einem gegenüber der perforierbaren Folie relativ stabilen und elastischen Material besteht. Der Behälter hat nach einer bevorzugten Ausführungsform die Form eines Napfes, wobei die den Öffnereinheiten zugewandte Fläche plan ausgebildet ist und aus perforierbarem Material besteht ; die restliche Wandung des Behälters kann durchsichtig oder pigmentiert sein.

Bei der bevorzugten Ausführungsform wird das Öffnen der perforierbaren Folie durch die Öffnereinheit dadurch bewerkstelligt, daß die vorzugsweise napfförmigen Behälter durch Fingerdruck auf die Öffnereinheiten zubewegt werden und die spitze oder dornartige Elemente aufweisenden Öffnereinheiten die perforierbare Folie durchdringen.

Nach weiteren Ausführungsform a sind auf der Gehäuserückseite ebenfalls Öffnungen vorgesehen, durch welche die Öffnereinheiten derart betätigbar sind, daß sie mit ihren spitzen oder dornartigen Elementen die perforierbare Folie der Wirkstoffbehälter durchdringen, wobei in diesem Fall die Behälter unverschiebbar im Gehäuse aufgenommen sind. So können u.a. auch Vorteilhafterweise bei Verdunstungsvorrichtungen alle Gehäuseteile gemeinsam in einem einzigen Arbeitsgang hergestellt werden. Die Verdunstungsvorrichtung läßt sich auf einfache und gefahrlose Weise zusammensetzen, wobei lediglich durch Aufeinanderklappen der einzelnen Gehäuseteile nach vorangehendem Einsetzen der Wirkstoff-Behälter die Verdunstungsvorrichtung betriebsfähig ist.

Im folgenden werden bevorzugte Ausführungsformen der Verdunstungsvorrichtung anhand der Zeichnung zur Erläuterung weiterer Merkmale beschrieben. Es zeigen :

Figur 1 eine Schnittansicht einer ersten Ausführungsform der Verdunstungsvorrichtung,

Figur 2 eine Perspektivansicht der Verdunstungsvorrichtung gemäß Fig. 1,

Figur 3 eine Schnittansicht einer zweiten Ausführungsform der Verdunstungsvorrichtung,

Figur 4 eine weitere Ausführungsform einer Verdunstungsvorrichtung, teilweise aufgebrochen,

Figur 5 eine Schnittdarstellung der Verdunstungsvorrichtung nach Fig. 4,

Figur 6 eine Schnittdarstellung durch eine weitere Ausführungsform einer Verdunstungsvorrichtung,

Figur 7 eine Schnittdarstellung einer weiteren Ausführungsform der Verdunstungsvorrichtung,

Figur 8 eine Draufsicht auf die Ausführungsform nach Fig. 7, und

Figur 9 eine schematische Schnittdarstellung einer gegenüber Fig. 7 abgewandelten Ausführungsform der Verdunstungsvorrichtung.

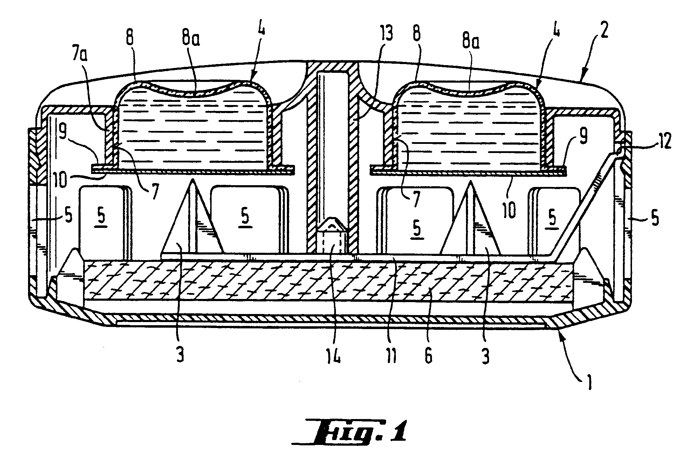

Die nachfolgend unter Bezugnahme auf die Fig. 1 bis 9 beschriebene Verdunstungsvorrichtung weist jeweils ein Gehäuse auf, das aus einem schalenförmigen oben offenen Gehäuseunterteil 1 und einem die Mündung des Gehäuseunterteils 1 verschließbaren, unten offenen Gehäuseoberteils 2 besteht. In dem Gehäuse ist jeweils eine Öffnereinheit 3 und wenigstens ein Behälter 4 für flüchtige Wirkstoffe, beispielsweise Insektizide, vorgesehen. Die Gehäuseteile 1, 2 sind jeweils in einem einfachen, lediglich aus einem Unter- und Oberteil bestehenden Formwerkzeug durch Urformen, vorzugsweise aus Kunststoff, hergestellt. Die Gehäuseteile 1, 2 sind um eine senkrechte Achse zueinander verdrehbar zusammengesteckt, um durch Verdrehen des Gehäuseoberteiles 2 gegenüber dem Gehäuseunterteil 1 im Gehäusemantel angeordnete Durchbrüche oder Öffnungen 5 mehr oder weniger schließen bzw. öffnen zu können. Die Durchbrüche 5 ermöglichen den Austritt von aus dem oder den Behältern 4 innerhalb des Gehäuses ausfließenden Wirkstoffen in die Umgebung der Verdunstungsvorrichtung.

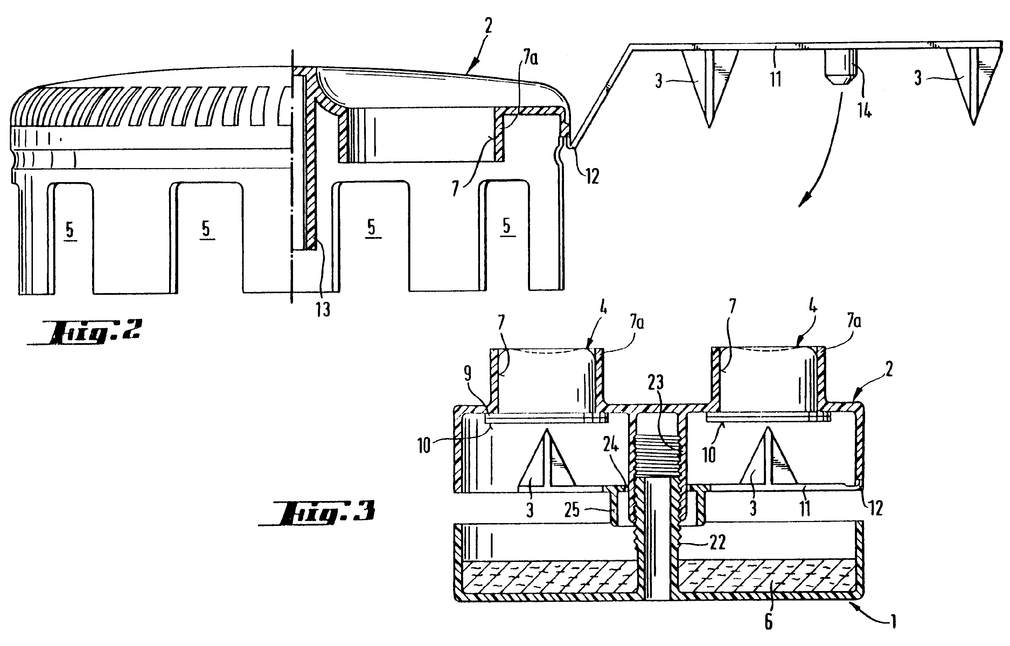

Die Verdunstungsvorrichtung weist ein saugfähiges Element 6 auf, das vorzugsweise aus Zellstoff besteht und dazu dient, die aus dem Behälter 4 austretenden Wirkstoffe aufzusaugen und derart zu speichern, daß die Wirkstoffe allmählich durch die Durchbrüche 5 gegenüber der Atmosphäre freigegeben werden können. Vorzugsweise ist das die Wirkstoffe aufsaugende Element 6 im Gehäuseunterteil 1 angeordnet, wie aus Fig. 1 hervorgeht. Im Gehäuseoberteil 2 sind mehrere, zur Drehachse des Gehäuses exzentrisch angeordnete und parallel verlaufende Öffnungen 7 vorgesehen, die als Aufnahmeabschnitt für den oder die Behälter 4 dienen und in welche jeweils von der Mündungsseite her ein Behälter 4 eingesteckt ist. Die Wirkstoffbehälter 4 bestehen vorzugsweise aus jeweils einem napfförmigen Hüllenteil 8, z.B. aus einem durchsichtigen Material, wie Kunststoff, oder aus einer pigmentierten Folie. Das Hüllenteil 8 weist einen angeformten, nach außen gerichteten Rand oder Kragen 9 und einen an dem Rand 9 gasdicht befestigten Boden 10 auf, der aus einem perforierbaren Material, vorzugsweise einer Metallfolie besteht und die der zugehörigen Öffnereinheit 3 zugewandte Fläche des Behalters 4 bildet. Der Boden 10 ist vorzugsweise plan ausgebildet. Die durch Öffnungen 7 definierten Aufnahmeabschnitte für jeden Behälter 4 ergeben sich bei der Ausführungsform nach Fig. 1 und 3 durch am Gehäuseoberteil 2 ausgebildete Flanschabschnitte 7a, die etwa parallel zur Gehäuseachse verlaufend vom Gehäuseoberteil 2 in Richtung auf das Gehäuseunterteil 1 gerichtet sind oder umgekehrt, wie dies bei den Ausführungformen nach Fig. 1 und 3 dargestellt ist. Der Durchmesser der vorzugsweise kreisförmigen Flansche 7a ist geringfügig größer gewählt als der entsprechende Außendurchmesser des in den Aufnahmeabschnitt 7 eingesetzten Hüllenteils 8, wobei der Rand 9 jedes Behälters 4 einen Durchmesser hat, der größer als der Durchmesser der Flansche 7a gewählt ist, so daß jeder Behälter 4 durch den Rand 9 an einer Verlagerung aus dem Aufnahmeabschnitt 7 und damit aus dem Gehäuse heraus gehindert ist.

Unterhalb jedes Aufnahmeabschnitts 7 und damit unterhalb jedes in den zugehörigen Aufnahmeabschnitt 7 eingesetzten Behälters 4 ist eine Öffnereinheit 3 angeordnet, die aus wenigstens einem spitz zulaufenden oder dornartigen Element gebildet ist. Die Öffnereinheit oder die Öffnereinheiten 3 sind an einem gemeisamen, streifenförmig und diametral zum Gehäuse verlaufenden Träger 11 angeformt, wobei ein Endteil des Trägers 11 mittels eines Filmscharniers 12 oder dergleichen am Gehäuseoberteil 2 angelenkt ist. Gemäß Fig. 1 und 2 ist der die Öffnereinheit oder Öffnereinheiten 3 lagernde Träger 11 vom Gehäuseoberteil radial abstrebend hergestellt, so daß die Unformung des Gehäuseoberteils 2 und des Trägers 11 gemeinsam in einem lediglich aus einem Ober- und Unterteil bestehenden Formwerkzeug erfolgen kann.

Bei der in Fig. 1 gezeigten Ausführungsform ist ein vom Gehäuseoberteil 2 von seiner Innenseite abstrebender, zum Gehäuseunterteil 1 verlaufender und zur Gehäuseachse bzw. Gehäusedrehachse koaxial angeordneter Rohrstutzen 13 vorgesehen, in welchen ein vom Träger 11 abstehender Lagerzapfen 14 mindestens mit Haftreibung gesichert so eingreift, daß sich der Träger 11 an der Mündung des Rohrstutzens 13 abstützt. Der Träger 11 kann in der Gebrauchslage auch durch angeformte Rastelemente gesichert sein. Anstelle eines einzigen geweils eine Öffnereinheit 3 bildenden dornartigen Elementes können auch mehrere derartige dornartige Elemente für jeweils einen Behälter 4 vorgesehen sein, wobei jede Gruppe derartiger dornartiger Elemente in der beschriebenen Weise vom Träger 11 unterhalb des eingesetzten Behälters 4 im Gehäuse gelagert ist. Nach einer bevorzugten Ausführungsform endet jedes Element jeder Öffnereinheit 3 in einer Meißelschneide.

Bei der Verdunstungsvorrichtung nach Fig. 3 ist das Gehäuseorberteil 2 vom Gehäuseunterteil 1 mittels eines zentral angeordneten Schraubentriebes abhebbar ausgebildet, so daß das Gehäuse mehr oder weniger geöffnet werden kann. Vom Gehäuseteil 1 steht zu diesem Zweck ein hohler Gewindezapfen 22 und vom Gehäuseoberteil 2 eine Gewindemuffe 23 ab. Der Träger 11 weist eine Bohrung 24 auf, in welche die Gewindemuffe 23 mit Bewegungsspiel eingreitf. Koaxial zur Bohrung 24 ist am Träger 11 ein Rohrstutzten 25 angeformt, der sich auf dem saugfähigen Element 6 abstützt.

Die Behälter 4 sind in die am Gehäuseoberteil 2 angeformten, nach außen (Fig. 3) abstehenden Rohrstutzen oder Flansche 7a von der Innenseite des Gehäuseoberteiles 2 her eingesetzt. Dabei ist ersichtlich, daß in die Verdunstungsvorrichtung gemäß der Erfindung entweder nur ein Behälter 4 einsetzbar oder auch zwei oder mehr derartige Behälter 4 einsetzbar sind, die gegebenenfalls auch exzentrisch vorgesehen sind. Zum Öffnen der Behälter 4 ist der betreffende Behälter 4 manuell gegenüber dem Flansch 7a bzw. dem Aufnahmeabschnitt 7 zu verschieben, und zwar in Richtung auf die zugehörige Öffnereinheit 3, bis die Spitze der zugehörigen Öffnereinheit oder des zugehörigen Dornes 3 den vorzugsweise durch eine perforierbare Folie gebildeten Boden 10 durchsticht, so daß die im Behälter 4 befindlichen Wirkstoffe austreten und auf das im Gehäuseunterteil 1 befindliches saugfähige Element 6 gelangen. Das saugfähige Element 6 in Form einer Schicht, beispielsweise aus Zellstoff, saugt die Wirkstoffe auf und ermöglicht eine allmähliche Verdunstung derselben an die Atmosphäre. Die Verwendung einer perforierbaren Folie als Boden 10 der Behälter 4 hat zum Vorteil, daß die Wirkstoffe vollständig aus dem Behälter nach Öffnung des Bodens 10 austreten und ein Wiederverschließen der durch die Öffnereinheit 3 gebildeten Öffnung oder Öffnungen nach Aufhebung des beispielsweise durch einen Finger, auf den Behälter 4 ausgeübten Druck ausgeschlossen ist. Damit ist ein vollständiges Entleeren jedes Behälters 4 sichergestellt. Wenn das napfförmige Hüllenteil 8 aus einem durchsichtigen Material hergestellt ist, ist es dem Benutzer der Verdunstungsvorrichtung möglich, den Füllzustand jedes Behälters 4 optisch zu erfassen und im Bedarfsfall nacheinander die noch ungefüllten Behälter in der beschriebenen Weise zur Freigabe der darin befindlichen Wirkstoffe zu öffnen. Da zum Zwecke der Öffnung der Behälter 4 dieselben mit dem Finger gegenüber dem jeweiligen Aufnahmeabschnitt 7 in Richtung auf die zugeordnete Öffnereinheit 3 zu verschieben sind und dann auf dem betreffenden Behälter 4 ein Druck zur Perforation des Bodens 10 ausgeübt werden muß. muß für das napfförmige Hüllenteil 8 ein Material gewählt werden, dessen Festigkeit so ausreichend ist. daß eine Beschädigung des Hüllenteils 8 während des Öffnungsvorganges ausgeschlossen ist. Wie aus der vorstehenden Beschreibung hervorgeht, soll lediglich der Boden 10 mittels der dornartigen Elemente bzw. Öffnereinheiten 3 perforiert werden, der restliche Teil jedes Behälters 4 soll dagegen möglichst unverformbar sein und den zu Öffnungszwecken ausgeübten Kräften standhalten. Dies bedeutet, daß der napfartige Hüllenteil 8 eine gegenüber der von der zugehörigen Öffnereinheit 3 durchtrennbaren, den Boden 10 bildenden Folie eine um so viel größere Formsteifigkeit haben muß, daß lediglich mit dem napfartigen Hüllenteil 8 die zum Öffnen der Folie erforderliche Druckkraft übertragbar ist.

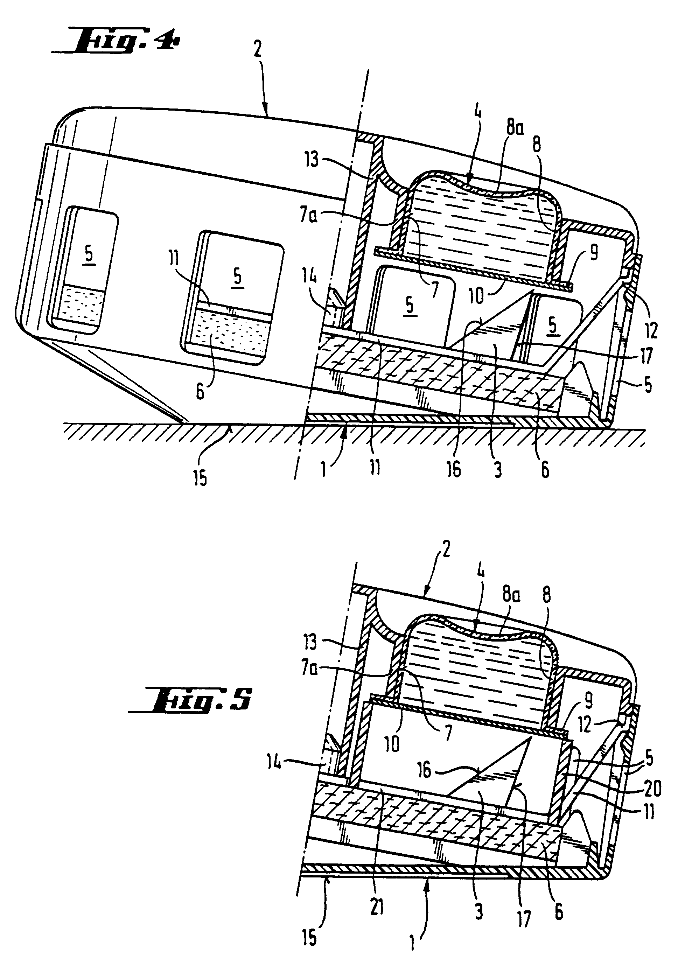

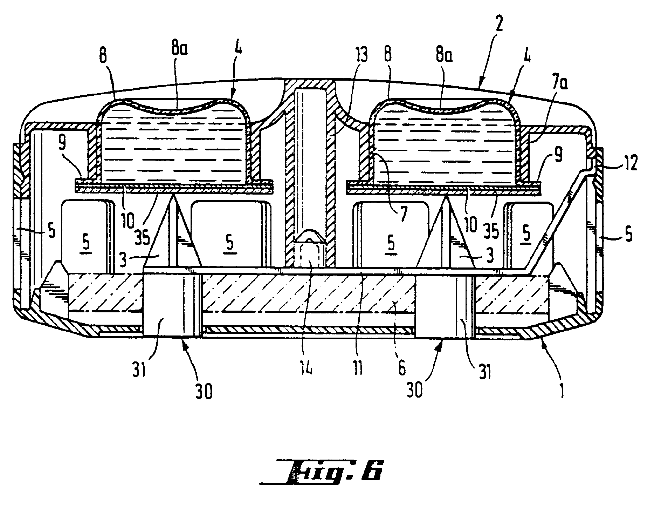

In den Fig. 4 und 5 sind weitere Ausführungsformen der Verdunstungsvorrichtung für Insektizide, Duftstoffe und/oder ander flüchtige Wirkstoffe dargestellt. Bei diesen Ausführungsformen ist je Behälter 4 als Öffnereinheit 3 ein dornartiges Element auf dem Träger 11 gelagert. Der Träger 11 kann dabei in der Gebrauchslage durch angeformte Rasterelemente gesichert sein. Das Gehäuse weist ein Gehäuseunterteil 1 mit geneigter Basisseite 15 auf, während das saugfähige Element 6. der Träger 11 und der Boden 10 in einer zueinander weitgehend parallelen Anordnung vorgesehen sind, wie dies bereits aus den Fig. 1 bis 3 ersichtlich ist. Der Träger 11 lagert als Öffnereinheit 3 jeweils eine dornartiges Element, wobei die Spitze dieses dornartigen Elementes dem Rand 9 benachbart liegend angeordnet ist und jedes dornartige Element eine dem Rand 9 abgewandte Schneidenseite 16 als Spanfläche und eine weitere Schneidenseite 17 als Freifläche aufweist. Das Öffnen des in Fig. 4 rechts dargestellten Behälters 4 erfolgt auf gleiche Weise wie dies vorstehend erläutert wurde, nämlich dadurch, daß mittels eines Fingers eine Druckkraft auf den Behälter 4 ausgeübt wird und dieser in Richtung auf die zugehörige Öffnereinheit 3 verlagert wird, so daß die Spitze der Öffnereinheit 3 den Boden 10 perforiert und den Austritt des im Behälter 4 befindlichen Wirkstoffes ermöglicht, infolgedessen die Wirkstoffe aus dem Behälter 4 austreten und von dem saugfähigen Element 6 aufgenommen werden. Zur Erleichterung des Öffnungsvorganges und insbesondere zur verkantungsfreien Verschiebung jedes Behälters 4 in Richtung auf seine zugehörige Öffnereinheit 3 ist das napfförmige Hüllenteil 8 mit einer Einwölbung 8a versehen, wodurch der Behälter 4 mit dem Finger einer Hand sicher in das Gehäuse bzw. in die Verdunstungsvorrichtung eingedrückt werden kann; die Einwölbung 8a bewirkt ferner eine Versteifung des Behälters 4 auf solche Weise, daß unmittelbar über den Behälter 4 die zum Öffnen der den Boden 10 bildenden Folie erforderliche Kraft ausgeübt werden kann.

Bei der in Fig. 5 gezeigten Ausführungsform ist jedem Behälter 4 mit dem unter Bezugnahme auf Fig. 1 bis 4 beschriebenen Aufbau ein mit der unteren Stirnseite auf dem Element 6 dicht schließend anliegender Ringkörper 20 zugeordnet, der an dem Träger 11 integriert ausgebildet ist. Der zylindrische Ringkörper 20 weist einen diametral gerichteten, angeformten Steg 21 auf, an dem innerhalb des Ringkörpers 20 die Öffnereinheit 3 angeformt ist. Der lichte Querschnitt des zylindrischen Ringkörpers 20 ist geringfügig kleiner als der lichte Querschnitt des durch die Folie gebildeten Bodens 10 des Behälters 4 und größer als der lichte Querschnitt des zylindrischen Aufnahmeabschnitts 7. Die Höhe der Öffnereinheit 3 ist kleiner als die Höhe des Ringkörpers 20 gewählt, d.h. die Öffnereinheit 3 bzw, das die Öffnereinheit 3 ergebende dornartige Element 3 ragt nicht über den Ringkörper 20 hinaus, so daß beim Eindrücken des Behälters 4 in das Gehäuse zum Zwecke des Öffnens des Bodens 10 die den Boden 10 bildende Folie über ihren gesamten Umfang innenseitig des Ringkörpers 20 sich dicht schließend am Ringkörper 20 anlegt und dann erst von der Öffnereinheit 3 durchdrungen bzw. perforiert wird. Dadurch ist sichergestellt, daß der aus dem Behälter 4 austretende Wirkstoff nicht seitlich in das Gehäuseoberteil oder Gehäuseunterteil abfließt, was bei den Ausführungsformen nach Fig. 1 bis 4 bei einer Schrägstellung der Verdunstungsvorrichtung möglich wäre, sondern durch den Ringkörper 20 in Richtung auf das saugfähige Element 6 geleitet wird. Duch die Zurückversetzung der Öffnereinheit 3 gegenüber dem Ringkörper 20 wird sichergestellt, daß die den Boden 10 bildende Folie des Behälters 4 durch die Öffnereinheit 3 erst dann perforiert wird, wenn der Boden 10 mit seinem Rand 9 dicht auf dem Ringkörper 20 aufliegt und damit die Wirkstoffe nicht unter Umgehung des Ringkörpers 20 in das Gehäuse austreten können, sondern in Richtung auf das saugfähige Element geführt werden.

Bei der Ausführungsform nach Fig. 5 wird durch das Ausüben einer Kraft mittels eines Fingers auf die Einwölbung 8a oder auf die aus dem zugehörigen Aufnahmeabschnitt 7 herausragende Wandung des napfförmigen Hüllenteils 8 erreicht, daß sich der Boden 10 mehr oder weniger stark in Richtung auf die Öffnereinheit 3 wölbt, wodurch das Perforieren des Bodens 10 durch die Öffnereinheit 3 erreicht wird. Der in Fig. 5 zwischen dem Boden 10 und der Öffnereinheit 3 gezeigte Abstand wird durch die auf den Behälter 4 ausgeübte Kraft so weit reduziert, daß die Spitze der Öffnereinheit 3 durch den Boden 10 hindurchdringt.

Unter Bezugnahme auf Fig. 6 wird nachfolgend eine weitere Ausführungsform der Verdunstungsvorrichtung beschrieben, die im wesentlichen gleichen Aufbau hat, wie die Verdunstungsvorrichtung nach den Fig. 1 bis 5. Bei der Ausführungsform nach Fig. 6 ist allerdings vorgesehen, daß im Gegensatz zu den vorher beschriebenen Ausführungsformen nicht der Behälter 4 in Richtung auf die zugehörige Öffnereinheit 3 zum Öffnen des folienartigen Bodens 10 zu verschieben ist, sondern daß demgegenüber jede Öffnereinheit 3 über eine zugehörige, im Gehäuseunterteil 1 ausgebildet Öffnung 30 derart betätigbar ist, daß die Spitze der betreffenden Öffnereinheit 3 in den Boden 10 des Behälters 4 verlagert werden kann. Jeder Aufnahmeabschnitt 7 ist in der Weise ausgebildet, wie dies unter Bezugnahme auf Fig. 1 beschrieben ist, während die Öffnereinheit 3 gegenüber Fig. 1 auf solche Weise abgewandelt ist, daß jedes spitze bzw. dornartige Element, das Teil Öffnereinheit ist oder die Öffnereinheit bildet, einen vom eingesetzten Behalter weglaufenden Verlängerungsabschnitt 31 aufweist, der innerhalb der im Gehäuseunterteil 1 ausgebildeten, zugehörigen Öffnung 30 zu liegen kommt und durch Fingerbetätigung die Verlagerung jeder einzelnen Öffnereinheit 3 in Richtung auf den Behälter 4 ermöglicht. Die Öffnereinheiten 3 sind dabei in gleicher Weise am Träger 11 befestigt, wie dies vorstehend beschrieben ist, wobei der Träger 11 jedoch eine gewisse Elastizität haben muß, um die Verlagerung der einzelnen Öffnereinheiten 3 unabhängig voneinander in Richtung auf den Behälter 4 zu ermöglichen. Bei der Ausführungsform nach Fig. 6 kann das saugfähige Element in Form einer Schicht im Gehäuseunterteil 1 vorgesehen sein, wie dies unter Bezugnahme auf Fig. 6 gezeigt und beschrieben ist, wobei die das saugfähige Element 6 bildende Schicht zur Durchführung der Verlängerungsabschnitte 31 Ausnehmungen aufzuweisen hat.

Somit ist das Element 6 in gleicher Weise bei der Ausführungsform nach Fig. 6 vorgesehen, wie dies in Fig. 1 gezeigt ist, besitzt jedoch mehrere, der Querschnittsfläche der Verlängerungen 31 entsprechende Aussparungen oder Öffnungen, so daß die Verlängerungen 31 von der zugehörigen Öffnereinheit 3 in Richtung auf das Gehäuseunterteil 1 durch das saugfähige Element 6 sich bis zur Öffnung 30 im Gehäuseunterteil 1 erstrecken können und damit von außen zum Öffnen des Bodens 10 des betreffenden Behälters 4 betätigbar sind. Das Element 6 ist in Fig. 6 nur strichliert eingetragen. Nach einer weiteren Ausführungsform ist bei einer Verdunstungsvorrichtung gemäß Fig. 6 das saugfähige Element 6 nicht vorgesehen ; stattdessen ist die Unterseite jedes Bodens 10 jedes Behälters 4 mit einer verhältnismäßig dünnen Schicht 35 versehen, die eine mit der Saugfähigkeit des Elementes 6 vergleichbare saugfähige Eigenschaft hat. Die Schicht 35 hat solche Stärke, daß ein Perforieren des folienartigen Bodens 10 mittels der Öffnereinheiten 3 gewährleistet und ein Wiederverschließen der hergestellten Perforation durch die Schicht 35 selbst verhindert ist. Bei dieser Ausführungsform ist sichergestellt, daß der aus dem betreffenden Behälter 4 austretende Wirkstoff über die durch die zugehörige Öffnereinheit 3 hergestellte Perforation in die saugfähige Schicht 35 zu der dann erwünschten Verdunstung eintritt, wobei infolge des direkten Kontaktes zwischen der Schicht 35 und dem Wirkstoff im Behälter 4 über die durch die zugehörige Öffnereinheit 3 hergestellte Öffnung im Boden 10 die Schicht 35 kontinuierlich Wirkstoff aus dem Behälter 4 aufnimmt und zur Verdunstung an die Atmosphäre freigibt.

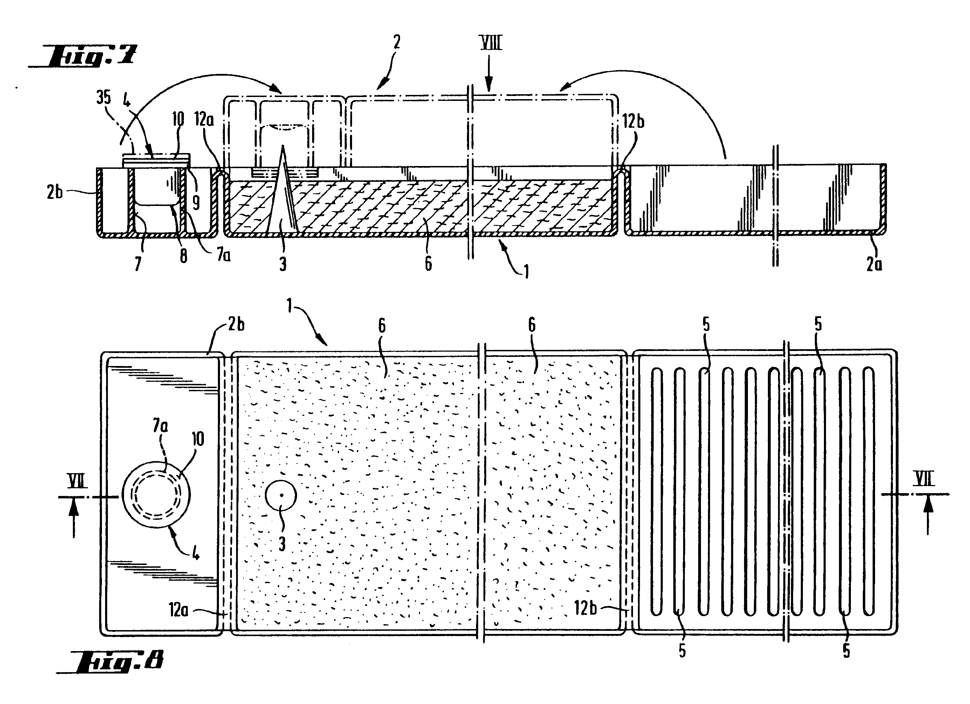

Eine weiter abgewandelte, Ausführungsform der Verdunstungsvorrichtung wird nachfolgend unter Bezugnahme auf die Fig. 7 bis 9 beschrieben. Bei dieser Ausführungsform besteht das Gehäuse aus einem Gehäuseunterteil 1 und einem zweiteiligen Gehäuseoberteil, wobei das eine Gehäuseoberteil mit 2a und das andere, den Aufnahmeabschnitt 7 für den Behälter 4 enthaltende Gehäuseoberteil mit 2b bezeichnet ist.

Beide Gehäuseoberteile 2a. 2b sind an jeweils zueinander abgewandten Endkanten des Gehäuseunterteils 1 angelenkt, vorzugsweise mittels Filmscharnieren 12a und 12b, wie aus Fig. 7 hervorgeht, wobei das in Fig. 7 linke Filmscharnier 5 zur Befestigung des Gehäuseoberteils 2b am Gehäuseunterteil 1 und das in Fig. 7 rechte Filmscharnier 12b zur Anlenkung des Gehäuseoberteils 2a am Gehäuseunterteil 1 dient. Der Aufnahmeabschnitt 7 besteht wie bei den vorstehend beschriebenen Ausführungsformen aus Flanschabschnitten 7a, die bei einem weitgehend zylindrischen bzw. napfförmigen Hüllenteil 8 ebenfalls zylindrische Form haben und wobei der Innendurchmesser des Flansches 7a geringfügig größer als der Außendurchmesser des napfförmigen Hüllenteils 8 gewählt ist. Die Behälter 4 haben im wesentlichen gleiche Form, wie dies unter Bezugnahme auf Fig. 1 bis 6 beschrieben ist, d. h. einen seitlich abstehenden Rand 9, der sich seitlich über den Flansch 7a hinweg erstreckt und als Anschlag gegen eine Bewegung des Behälters 4 aus dem Aufnahmeabschnitt 7 in Richtung aus dem Gehäuse heraus dient. Die zugehörige Öffnereinheit oder Öffnereinheiten 3 sind im Gehäuseunterteil 1 ausgebildet und bewirken eine Perforation des Bodens 10 des Behälters 4, sobald nach Schließen des Gehäuses, d. h. nach Umklappen der beiden Gehäuseteile 2a, 2b, der betreffende Behälter 4 innerhalb des Aufnahmeabschnittes 7 nach unten in Richtung auf die Öffnereinheit 3 verlagert wird. Zum Öffnen bzw. Perforieren jedes Behälters 4 ist im wesentlichen der gleiche Vorgang auszuführen, wie dies unter Bezugnahme auf Fig. 1 erläutert ist. Wie aus Fig. 7 ersichtlich ist, befindet sich im Gehäuseunterteil 1 das saugfähige Element 6 in Form einer Schicht, wobei im Bereich der Öffnereinheit 3 das Element 6 eine entsprechende Aussparung aufweist ; bei einem relativ lockeren Material für das Element 6 ist gegebenenfalls das Vorsehen einer entsprechenden Aussparung für die Öffnereinheit 3 überflüssig, vielmehr wird das Element 6 über die Öffnereinheit in Form eines dornartigen Elementes 3 in das Gehäuseunterteil eingesetzt, wobei sich das dornartige Element 3 durch das Element 6 hindruchbohrt und die in Fig. 7 gezeigte Lage einnimmt. Wie unter Bezugnahme auf Fig. 6 beschrieben ist. kann auch ein Behälter 4 mit an seiner Unterseite ausgebildeter saugfähiger Schicht 35 vorgesehen sein, wobei dann im Gegensatz zu Fig. 7 das Element 6 entfällt.

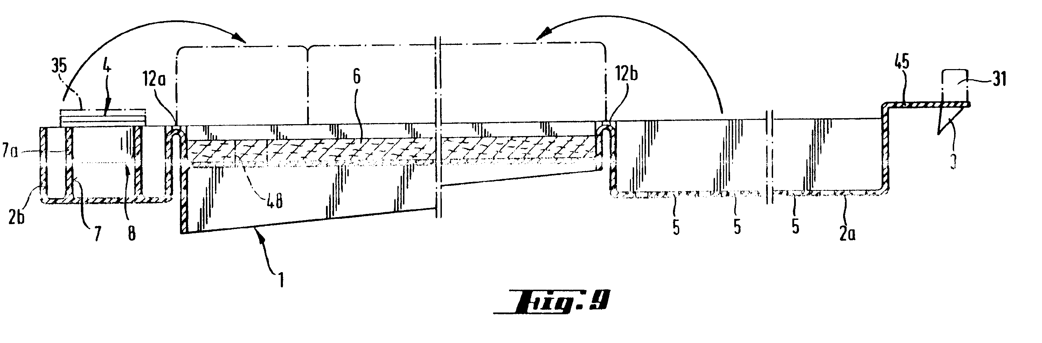

Wenn die Gehäuseoberteile 2a und 2b in die in Fig. 7 strichpunktiert dargestellte Lage verschwenkt sind. rasten sie vorzugsweise mittels nicht dargestellter Rastnocken am Gehäuseunterteil 1 ein, so daß ein selbsttätiges und unbeabsichtigtes Aufklappen der Gehäuseoberteile 2a. 2b ausgeschlossen ist. Das Verdunsten des entweder in das saugfähige Element 6 eingeflossenen Wirkstoffes oder von der Schicht 35 aufgenommenen Wirkstoffes wird durch Durchbrüche 5 gewährleistet, die vorzugsweise im Gehäuseoberteil 2a ausgebildet sind und gegebenenfalls auch noch zusätzlich im Gehäuseoberteil 2b vorgesehen sein können. Eine Draufsicht auf die aufgeklappte Verdunstungsvorrichtung entsprechend der voll ausgezeichneten Anordnung nach Fig. 7 zeigt Fig. 8. Anstelle eines einzigen Behälters 4 können auch bei dieser Ausführungsform zwei oder mehrere derartige Behälter und entsprechend viele Aufnahmeabschnitte 7 vorgesehen sein.

Eine gegenüber Fig. 7 abgewandelte Ausführungsform der Verdunstungsvorrichtung zeigt Fig. 9. Bei dieser Ausführungsform ist die Öffnereinheit 3 nicht im Gehäuseunterteil 1 angeformt, sondern - wie in Fig. 9 dargestellt - am Gehäuseunterteil 2a ausgebildet. Die Öffnereinheit oder Öffnereinheiten 3 sind mittels einer sich vom Gehäuseoberteil 2 abstehenden Lasche 45 ausgebildet, wobei die Lasche in dem in Fig. 9 gezeigten aufgeklappten Zustand sich in Axialrichtung des Gehäuses erstreckt und wobei die Öffnereinheit 3 in Form eines dornartigen Elementes im wesentlichen nach unten absteht. Wenn das Gehäuseoberteil 2a in die Gebrauchsstellung umgeschwenkt wird, wie dies in Fig. 7 strichpunktiert dargestellt ist, anschließend auch das Gehäuseoberteil 2b in die Gebrauchsstellung umgeklappt wird, wie dies ebenfalls in Fig. 7 strichpunktiert dargestellt ist, kommt die Öffnereinheit 3 unterhalb des Behälters 4 zu liegen und der Behälter 4 kann durch Ausübung einer Kraft auf sein napfförmiges Hüllenteil 8 in Richtung auf die Öffnereinheit 3 zur Perforation des Bodens verschoben werden. Auch bei dieser Ausführungsform kann das saugfähige Element 6 entfallen, wenn ein Behälter 4 verwendet wird, an dessen Bodenfläche 10 eine saugfähige Schicht 35 angeordnet ist. Schließlich kann bei der in Fig. 9 gezeigten Ausführungsform die Öffnereinheit 3 mit einer zum dornartigen Element entgegengesetzten Verlängerung 31 versehen sein, so daß nach dem Umklappen des Gehäuseoberteils 2a aus der in Fig. 9 gezeigten Lage in die in Fig. 7 gezeigte Position die Verlängerung 31 in einer für die Verlängerung 31 vorgesehenen und gestrichelt in Fig. 9 angedeutete Öffnung 48 zu liegen kommt. Damit läßt sich jeder Behälter 4 dadurch öffnen, daß die Öffnereinheit über die Öffnung 48 im Boden des Gehäuseunterteils 1 derart betätigt wird. daß das dornartige Element in den Boden 10 des betreffenden Behälters 4 verlagert wird, wie dies unter Bezugnahme auf Fig. 6 erläutert ist.

Soweit in Verbindung mit dem Träger 11 ein Ringkörper 20 verwendet wird, wie dies in Bezug auf Fig. 5 beschrieben ist, wird bei gleichzeitiger Benutzung eines saugfähigen Elementes 6, das in das Gehäuseunterteil 1 eingesetzt ist, die Anordnung derart getroffen, daß der Ringkörper 20 mit seiner unteren Kante weitgehend auf dem Element 6 aufliegt, so daß nach Öffnen des folienartigen Bodens 10 durch die zugehörige Öffnereinheit 3 sichergestellt ist, daß die Wirkstoffe nur und nur auf das Element 6 auffließen. Es ist ersichtlich, daß der Ring-

EP 0 094 499 B2

körper 20 auch bei den Ausführungsformen nach Fig. 1 bis 4 und 6 bis 9 vorgesehen sein kann, um ein seitliches Abfließen der aus dem Behälter 4 austretenden Wirkstoffe in Richtung auf die Gehäuseinnenwandung zu vermeiden.

Aus vorstehender Beschreibung ist ersichtlich, daß die Verdunstungsvorrichtung zur Aufnahme eines oder mehrerer, beispielsweise zweier Behälter für Wirkstoffe konzipiert sein kann. Die Verwendung mehrerer Behälter innerhalb einer Verdunstungsvorrichtung hat zum Vorteil, daß die Verdunstungsvorrichtung im gebrauchsfertigen Zustand über einen langen Zeitraum verwendbar ist. Das napfförmige Hüllenteil 8 kann dabei aus einem durchsichtigen Material bestehen, so daß ohne weiteres erkennbar ist, ob der Behälter mit Wirkstoff gefüllt oder nur teilweise gefüllt oder entleert ist. Andererseits kann das napfförmige Hüllenteil 8 auch aus einer pigmentierten Folie hergestellt sein, wobei jeweils darauf zu achten ist, daß das napfartige Hüllenteil 8 eine größere Formsteifigkeit besitzt als der durch eine Folie gebildete Boden 10, der das Hüllenteil 8 dichtend abschließt.

Der vorzugsweise vorgesehene Ringkörper 20 hat eine Höhe, die gleich, kleiner oder auch größer als die Höhe des dornartigen Elementes bzw, der Öffnereinheit 3 ist. Vorteilhaft wird die Höhe des Ringkörpers 20 so gewählt, daß der Rand 9 des Behälters 4 auf dem Ringkörper 20 aufliegt, bevor der Boden 10 durch die dornartigen Elemente bzw. Öffnereinheit 3 perforiert wird, also bevor die Wirkstoffe austreten.


**Patentansprüche**

1. Verdunstungsvorrichtung für Insektizide, Duftstoffe und/oder andere flüchtige Wirkstoffe, mit einem Durchbrechungen enthaltenden Gehäuse, welches mindestens einen Aufnahmeabschnitt (7), in den ein Behälter (4) für die Wirkstoffe eingesetzt ist, und mindestens eine dem Behälter (4) zugeordnete Öffnereinheit (3) aufweist, wobei zwischen dem Behälter (4) und der zugehörigen Öffnereinheit (3) eine Relativbewegung zum Öffnen des Behälters (4) ausführbar ist, sowie gegebenenfalls mit einem die aus dem Behälter (4) austretenden Wirkstoffe aufnehmenden Element (6), **dadurch gekennzeichnet** , daß im Gehäuse (1, 2) im Bereich jedes Aufnahmeabschnitts (7) eine Öffnung vorgesehen ist, durch welche wenigstens ein Teil der Wandung (8) des in den Aufnahmeabschnitt (7) des Gehäuses (1, 2) eingesetzten einzigen Behälters (4) zur Gehäuseaußenseite freiliegend angeordnet ist, daß jeder Behälter (4) innerhalb des Aufnahmeabschnitts (7) in Richtung auf die zugehörige Öffnereinheit (3) verlagerbar vorgesehen ist, und daß jeder Behälter (4) einen seitlich abstehenden Kragen oder Rand (9) aufweist, dessen Außenabmessung größer als die des Aufnahmeabschnittes (7) gewählt ist, wobei der Kragen oder Rand (9) bei eingesetztem Behälter (4) im Gehäuseinneren zu liegen kommt.

2. Verdunstungsvorrichtung nach Anspruch 1, dadurch gekennzeichnet, daß wenigstens die zum Inneren des Gehäuses (1, 2) abgewandte Wandung(8) des Behälters (4) aus druchsichtigem oder pigmentiertem Material besteht.

3. Verdunstungsvorrichtung nach Anspruch 1 oder 2, dadurch gekennzeichnet, daß wenigstens zwei Aufnahmeabschnitte für jeweils einen Behälter (4) vorgesehen sind.

4. Verdunstungsvorrichtung nach einem der Ansprüche 1 bis 3, dadurch gekenzeichnet, daß der der Öffnereinheit (3) zugewandte Boden (10) jedes Behälters (4) eben ausgebildet ist.

5. Verdunstungsvorrichtung nach Anspruch 4, dadurch gekennzeichnet, daß der Boden (10) des Behälters (4) aus einem perforierbaren Material, z. B. einer Folie, besteht.

6. Verdunstungsvorrichtung nach einem der Ansprüche 1 bis 5, dadurch gekennzeichnet, daß jeder Aufnahmeabschnitt (7) durch einen zylindrischen Flansch (7a) gebildet ist, und daß der Flansch (7a) als Anschlag gegenüber dem Rand (9) eines Behälters vorgesehen ist.

7. Verdunstungsvorrichtung nach Anspruch 1 dadurch gekennzeichnet, daß der Boden (10) am Rand (9) gasdicht befestigt ist.

8. Verdunstungsvorrichtung nach einem der Ansprüche 1 bis 7, dadurch gekennzeichnet, daß jede Öffnereinheit (3) mindestens ein auf den Aufnahmeabschnitt (7) spitz zulaufendes, dornartiges Element aufweist.

9. Verdunstungsvorrichtung nach einem der Ansprüche 1 bis 8, wobei das Gehäuse aus einem Oberteil (2) und einem Unterteil (1) besteht, dadurch gekennzeichnet, daß der Aufnahmeabschnitt (7) im Gehäuseoberteil (2) ausgebildet ist und daß jede Öffnereinheit (3) am Oberteil (2) angelekt ist.

10. Verdunstungsvorrichtung nach wenigstens einem der Ansprüche 1 bis 9, dadurch gekennzeichnet, daß das saugfähige Element (6), welches die nach Öffnen bzw. Perforieren der Behälter (4) aus diesen austretenden Wirkstoffe aufnimmt, zwischen der Öffnereinheit (3) und dem Behälter (4) liegend angeordnet ist.

11. Verdunstungseinheit nach Anspruch 10, dadurch gekennzeichnet, daß das saugfähige Element durch eine saugfähige Schicht (35) gebildet ist, die an dem der Öffnereinheit (3) zugewandten Boden (10) vorgesehen ist.

12. Verdunstungsvorrichtung nach wenigstens einem der Ansprüche 1 bis 11, dadurch gekennzeichnet,

7

daß im Bereich jeder Öffnereinheit (3) ein ringförmiger Körper (20) vorgesehen ist, auf welchem der Boden (10) eines Behälters (4) aufliegt.

13. Verdunstungsvorrichtung nach einem der Ansprüche 1 bis 12, dadurch gekennzeichnet, daß die aus dem Gehäuse (1, 2) nach außen weisende und vom Aufnahmeabschnitt (8) umgebene Fläche der Wandung (8) jedes Behälters eine Einwölbung (8a) aufweist.

14. Verdunstungsvorrichtung nach einem der Ansprüche 1 bis 13, dadurch gekennzeichnet, daß die Wandung (8) des Behälters (4) die Gestalt eines Napfes hat.

## Claims

1. Evaporator device for insecticides, aromatic substances and/or other volatile active substances, with a housing comprising apertures and having at least one receiving portion (7) into which a container (4) for the active substances is inserted, and, associated with the container (6), at least one opener unit (3), the possibility being afforded of carrying out in order to open the container (4) a relative movement between the container (4) and the associated opener unit (3), and if necessary also comprising an element (6) for receiving the active substances emerging from the container (4), characterised in that there is in the housing (1, 2), in the region of each receiving portion (7) an aperture by which at least a part of the wall (8) of the single container (4) which is inserted into the receiving portion (7) of the housing (1, 2) is exposed to the outside of the housing, in that each container (4) is adapted for displacement within the receiving portion (7) and in the direction of the associated opener unit (3), and that each container (4) has a laterally projecting collar or rim (9), the outside dimensions of which are greater than those of the receiving portion (7), the collar or rim (9) being inside the housing when the container (4) is inserted.

2. Evaporator device according to Claim 1, characterised in that at least the walls (8) of the container (4) which are adverted from the interior of the housing (1, 2) consist of transparent or pigmented material.

3. Evaporator device according to Claim 1 or 2, characterised in that at least two receiving portions are provided to accommodate in each case one container (4).

4. Evaporator device according to one of Claims 1 to 3, characterised in that the bottom (10) of each container (4) which is towards the opener unit (3) is of flat construction.

5. Evaporator device according to Claim 4, characterised in that the bottom (10) of the container (4) consists of a perforable material, e.g. a film.

6. Evaporator device according to one of Claims 1 to 5, characterised in that each receiving portion (7) is constituted by a cylindrical flange (7a) and in that the flange (7a) is provided as an abutment in respect of the rim (9) of a container.

7. Evaporator device according to Claim 4, characterised in that the bottom (10) is fixed in gas-tight fashion on the rim (9).

8. Evaporator device according to one of Claims 1 to 7, characterised in that each opener unit (3) has at least one punch-shaped element which runs to a point towards the receiving portion (7).

9. Evaporator device according to one of Claims 1 to 8, the housing consisting of an upper part (2) and a bottom part (1), characterised in that the receiving portion (7) is constructed in the upper part (2) of the housing and in that each opener unit (3) is articulated on the upper part (2).

10. Evaporator device according to at least one of Claims 1 to 9, characterised in that the absorbent element (6) which receives the active substances emerging from the containers (4) after these have been opened or perforated, is disposed so that it lies between the opener unit (3) and the container (4).

11. Evaporator unit according to Claim 10, characterised in that the absorbent element is constituted by an absorbent lager (35) provided on the bottom (10) which is towards the opener unit (3).

12. Evaporator device according to at least one of Claims 1 to 11, characterised in that there is in the region of each opener unit (3) an annular member (20) on which rests, the bottom (10) of a container (4).

13. Evaporator device according to one of Claims 1 to 12, characterised in that the area of the wall (8) of each container which is directed outwardly and which is enclosed by the receiving portion (7) has in it a dimple (8a).

14. Evaporator device according to one of Claims 1 to 13, characterised in that the wall (8) of the container (4) is in the form of a cup.

## Revendications

1. Dispositif de vaporisation pour insecticides, matières odoriférantes et/ou autres matières actives volatiles, comportant un boîtier muni de perçages qui présente au moins une section réceptacle (7) dans laquelle est monté un réservoir (4) pour les matières actives et au moins un dispositif d'ouverture (3) de ce réservoir (4), un mouvement relatif étant réalisable entre le réservoir seul (4) et le dispositif d'ouverture (3), en vue de l'ouverture du réservoir (4), le dispositif de vaporisation comportant aussi le cas échéant un élément (6) recevant les matières actives s'échappant du réservoir (4), caractérisé par le fait que le boîtier (1, 2) comporte, dans la zone de chaque section réceptable (7), une ouverture dans laquelle au moins une partie de la paroi (8) du réservoir (4) monté dans la section réceptacle (7) du boîtier (1, 2) est disposée librement par rapport au côté externe du boîtier par le fait que chaque réservoir (4) est mobile à l'intérieur de la section réceptacle (7) de manière à pouvoir être déplacé vers le dispositif d'ouverture (3) correspondant et par le fait que chaque réservoir (4) présente un collet ou rebord (9), débordant latéralement, dont la dimension extérieure est supérieure à celle de la section réceptacle (7), ledit collet ou rebord (9) venant s'appuyer à l'intérieur du boîtier lorsque le réservoir (4) est mis en place.

2. Dispositif de vaporisaiton selon la revendication 1, caractérisé par le fait que, sur le réservoir (4), au moins la paroi (8) éloignée de l'intérieur du boîtier (1, 2) est constituée en une matière transparente ou pigmentée.

3. Dispositif de vaporisation selon la revendication 1 ou 2, caractérisé par le fait qu'au moins deux sections réceptacles sont prévues pour chaque réservoir (4).

4. Dispositif de vaporisation selon l'une des revendications 1 à 3, caractérisé par le fait que, sur chaque réservoir (4), le fond (10) orienté vers le dispositif d'ouverture (3) est de forme plane.

5. Dispositif de vaporisation selon la revendication 4, caractérisé par le fait que le fond (10) du réservoir (4) est constitué en une matière perforable, par exemple une feuille.

6. Dispositif de vaporisation selon l'une des revendications 1 à 5, caractérisé par le fait que chaque section réceptacle (7) est constituée par une bride cylindrique (7a) et par le fait que cette bride (7a) est agencée de manière à servir de butée à l'égard du rebord (9) d'un réservoir.

7. Dispositif de vaporisation selon la revendication 4, caractérisé par le fait que le fons (10) est fixé sur le rebord (9) de manière étanche aux gaz.

8. Dispositif de vaporisation selon l'une des revendications 1 à 7, caractérisé par le fait que chaque dispositif d'ouverture (3) présente au moins un élément pointu à la manière d'un poinçon, vers la section réceptacle (7).

9. Dispositif de vaporisation selon l'une des revendications 1 à 8, le boîtier étant constitué par une partie supérieure (2) et par une partie inférieure (1), caractérisé par le fait que la section réceptacle (7) est réalisée dans la partie supérieure du boîtier (2) et que chaque dispositif d'ouverture (3) est articulé sur ladite partie supérieure (2).

10. Dispositif de vaporisation selon l'une des revendications 1 à 9, caractérisé par le fait que l'élément aspirant (6) qui reçoit après ouverture ou perforation des réservoirs (4) les matières actives s'échappant de ceux-ci, est placé entre le dispositif d'ouverture (3) et le réservoir (4).

11. Dispositif de vaporisation selon la revendication 10, caractérisé par le fait que l'élément aspirant est constitué par une couche poreuse (35) qui est prévue sur le fond (10) orienté vers le dispositif d'ouverture (3).

12. Dispositif de vaporisation selon l'une au moins des revendications 1 à 11, caractérisé par le fait que, dans la zone de chaque dispositif d'ouverture (3), il est prévu un corps annulaire (20) sur lequel s'appuie le fond (10) d'un réservoir (4).

13. Dispositif de vaporisation selon l'une des revendications 1 à 12, caractérisé par le fait que la surface de la paroi (8) de chaque réservoir qui est orientée vers l'extérieur du boîtier (1, 2) et qui est entourée par la section réceptacle (7), présente une concavité (8a).

14. Dispositif de vaporisation selon l'une des revendications 1 à 13, caractérisé par le fait que la paroi (8) du réservoir (4) a la forme d'un godet.

**_Fig. 1_**

Fig.2

Fig.3

**Fig. 4**

**Fig. 5**

*Fig. 6*

Fig.7

Fig.8

**Fig. 9**

EP 0 094 499 B2